**Europäisches Patentamt**

**European Patent Office**

**Office européen des brevets**

(11) Numéro de publication : **0 519 777 A1**

# (12) DEMANDE DE BREVET EUROPEEN

(21) Numéro de dépôt : **92401468.1**

(22) Date de dépôt : **27.05.92**

(51) Int. Cl.$^5$ : **A61K 7/48,** A61K 35/78

(30) Priorité : **20.06.91 FR 9107587**

(43) Date de publication de la demande :
**23.12.92 Bulletin 92/52**

(84) Etats contractants désignés :
**DE ES GB IT**

(71) Demandeur : **LABORATOIRES DE BIOLOGIE VEGETALE YVES ROCHER**
**La Croix des Archers**
**F-56200 La Gacilly (FR)**

(72) Inventeur : **Pinchon, Henri**
**343 rue de la Petite Motte**
**F-45160 Olivet (FR)**
Inventeur : **Saboureau, Alain**
**17 Allée du Berry**
**F-45560 St. Dénis en Val (FR)**

(74) Mandataire : **Kügele, Bernhard**
**NOVAPAT FRANCE 63 bis, boulevard Bessières**
**F-75017 Paris (FR)**

(54) **Formes galeniques à base de plantes fraiches ou sèches.**

(57)    La présente invention est relative à une composition sous forme de gel transparent ou d'émulsion transparente, contenant des plantes fraîches ou sèches, soit entières, soit en fragments, soit en grains ou particules, soit à l'état lyophilisé, à son procédé de préparation et à son utilisation en cosmétologie, en hygiène et en pharmacie.

Cette composition est caractérisée en ce que le gel ou l'émulsion est transparent, et en ce que les plantes ou le produit lyophilisé s'y trouvent en suspension.

Produit, forme galéniques contenant ladite composition. Utilisation de la composition en application externe ou interne en pharmacie, en cosmétologie ou en hygiène.

EP 0 519 777 A1

La présente invention concerne une composition à base de plantes, utilisable en cosmétologie, en hygiène ou en pharmacie.

L'invention concerne également un procédé de préparation de cette composition.

Depuis longtemps, on utilise des plantes en raison de leurs propriétés curatives ou antiseptiques ou stimulantes pour certaines fonctions physiologiques. C'est ainsi qu'il existe à l'heure actuelle, de nombreux médicaments, des produits d'hygiène, ainsi que des produits de cosmétologie à base de plantes.

Cependant, dans ces produits connus, il n'est pas possible d'apprécier la quantité, la qualité des plantes introduites, ni leur stabilité à l'oeil nu. Or, une telle appréciation permettrait notamment, de visualiser les éventuelles dégradations dues au stockage et au transport, par exemple, entre la préparation de la composition et sa vente au public. Cette appréciation présenterait un réel avantage, surtout en ce qui concerne les médicaments ou les dentifrices, et permettrait l'utilisation de tels produits dans les meilleures conditions.

C'est pourquoi la présente invention a pour but de proposer une composition à base de plantes, qui soit d'un usage facile et commode et qui permette d'apprécier la qualité, la quantité et la stabilité des plantes ou des éléments obtenus à partir des plantes, qui s'y trouvent.

A cet effet, la composition selon l'invention est caractérisée en ce qu'elle comprend un support transparent comportant un gel et/ou une émulsion, et des éléments solides obtenus à partir des plantes fraîches ou sèches, se trouvant en suspension dans ledit support.

Les éléments solives peuvent se présenter sous forme de grains ou particules obtenus à partir des plantes par broyage ou cryobroyage.

Alternativement, les éléments solides peuvent être des plantes entières ou des parties entières de ces plantes telles que des feuilles entières ou des pétales entiers.

Avantageusement, les éléments solides, qu'ils soient formés de plantes fraîches ou sèches sous forme intégrale ou broyée, sont revêtus d'une couche de protection. Ceci est particulièrement avantageux dans le cas où les éléments solides comprennent des plantes fraîches ou sèches utilisées dans leur intégralité, car grâce à la présence de cette couche de protection, lesdites plantes sont maintenues de façon homogène dans un milieu préservant toutes leurs vertus.

Cette couche de protection peut contenir des charges minérales ou organiques telles que : oxyde de fer, oxyde de zinc, oxyde de titane, oxyde de chrome, talc, silice, acides aminés, sucre, lécithine, soie, cholestérol.

Les éléments solides peuvent être obtenus également par lyophilisation de plantes.

Selon un mode de réalisation de l'invention, la composition contient de 0,05 à 20% en poids de plantes.

Selon un mode de réalisation de l'invention, la composition contient de 0,1 à 21% en poids d'un agent gélifiant.

Selon un mode de réalisation de l'invention, la composition contient un agent tensioactif dans des proportions allant de 5 à 50% en poids.

Les éléments solides peuvent être obtenus à partir de plantes d'une même espèce, ou bien à partir d'un mélange de plantes d'espèces différentes.

Pour une composition destinée à être absorbée par voie orale, les plantes peuvent être choisies dans le groupe comprenant :

- tamarin, artichaut, bourrache pour purifier les voies biliaires ;
- gentiane, quinquina pour stimuler l'appétit ;
- fucus, badiane, mélisse pour faciliter la digestion ;
- coquelicot, passiflore, tilleul, camomille pour traiter les troubles du sommeil ;
- cannelle, églantier, maté, thé pour les asthénies fonctionnelles, états de fatigue passagers,
- marron d'inde, petit houx, hamamélis, myrtille, cassis pour traitement de la circulation veineuse et capillaire ;
- artichaut, cerise, cassis, framboise pour faciliter l'élimination urinaire ;
- vanille, frêne, sureau, thé comme adjuvant des régimes amaigrissants ;
- ginseng pour obtenir un produit à propriétés vitaminiques et apportant des éléments minéraux.

Pour une composition destinée à être administrée par voie externe, les plantes utilisées peuvent être choisies dans le groupe comprenant :

- la Camomille (Anthémis nobilis, Matricaria chamomilla) ;
- le Bleuet (Centaurea cyanus) ;
- la Rose (Rosa rubra, Rosa centifolia) ;
- la Menthe (Mentha aquatica L., arvensis L., M.spicata var. crispa L., M. piperata L., M. viridis L.) ;
- la Réglisse (Glycyrrhiza glabra, glycyrrhiza uranacis) ;
- Immortelle (Helichrysum arenarium, Helichrysum staechas).

L'agent gélifiant peut être choisi dans le groupe comprenant :

- les galactomannanes ;

- les farines à base de céréales alimentaires ;
- la cellulose et ses dérivés ;
- les acides polyacryliques réticulés ;
- les silices ;
- la gélatine ;
- l'agar agar ;
- les gommes adragantes ou arabiques ;
- les alginates et les pectines.

L'agent tensioactif utilisé peut être choisi dans le groupe qui comprend les anioniques, les nonioniques, les cationiques et les amphotères.

La composition selon l'invention peut comprendre aussi un véhicule de l'élément actif de la plante, ce véhicule pouvant être un ou plusieurs glycols, éventuellement associés à un ou plusieurs additifs déterminés en fonction du mode d'utilisation de la composition.

L'invention concerne également un produit cosmétologique, tel que masques de beauté, compositions antirides, compositions exfoliantes, gels pour la douche et le bain, dentifrices, savons, obtenus à partir de la composition selon l'invention telle que définie ci-dessus, en y ajoutant au moins un adjuvant cosmétique tel que modificateurs de pH, parfums, agents conservateurs, agents anti-oxydants, agents colorants.

La composition selon l'invention est avantageusement préparée selon le procédé suivant.

On utilise comme produits de départ des plantes fraîches ou sèches. Ces plantes sont éventuellement broyées soit à température ambiante, soit à très basse température (cryobroyage). Les plantes ou fragments de plantes, sont alors traités par un procédé leur assurant une bonne conservation dans le temps. Ce traitement peut consister en un enrobage ou en une lyophilisation. Les produits solides obtenus sont alors introduits dans un support transparent, sous forme de gel ou d'émulsion dont la viscosité permet le maintien en suspension desdits éléments solides.

Outre les dispositions qui précèdent, l'invention comprend encore d'autres dispositions, qui ressortiront de la description qui va suivre, laquelle se réfère, d'une part, à un procédé de préparation d'un gel et/ou émulsion transparent conforme à l'invention et, d'autre part, à des exemples de compositions utilisables aussi bien en cosmétologie qu'en hygiène.

Il doit être bien entendu toutefois que les exemples décrits ci-après sont donnés uniquement à titre d'illustration de l'objet de l'invention, dont ils ne constituent en aucune manière une limitation.

Les exemples ci-après indiquent des compositions cosmétiques, pharmaceutiques ou d'hygiène qui comportent des adjuvants tels que parfums, conservateurs, glycols ou colorants. Les pourcentages sont exprimés en poids/poids.

Exemple I : gel hydratant

```
- Polyethylèneglycol (30) nonylphenyl éther :
  de 0,5 à 8%, de préférence            2,00%
- Polyethylèneglycol dodecyl éther :
  de 0,25 à 4%, de préférence           1,00%
- α-bisabolol :
  de 0,02 à 0,4%, de préférence         0,10%
- Conservateurs :
  de 0,1 à 1,6%, de préférence          0,40%
- Butanediol-1,3 :
  de 0,8 à 12%, de préférence           3,00%
- Acide alginique :
  de 0,5 à 10%, de préférence           2,50%
- L-Lysine (sol. à 50% aq.) :
  de 0,5 à 8%, de préférence            2,00%
- L-Arginine :
  de 0,07 à 1,2%, de préférence         0,30%
- Aminobutanol :
  de 0,05 à 0,8%, de préférence         0,20%
- Eau déminéralisée :
  qsp                                   100%
- Parfum :
  de 0,02 à 0,4%, de préférence         0,10%
- Pétales broyés de camomille et de menthe :
  de 0,2 à 4%, de préférence            1,00.
```

Ce produit se présente sous forme d'un gel transparent de pH 5 à 5,5 qui s'étale facilement. Sa viscosité est de 16000 à 25000 mPa. $s^{-1}$.

Exemple II : Gel exfoliant

```
- Acide polyacrylique :
  de 0,15 à 2,4%, de préférence         0,60%
- Gomme xanthane :
  de 0,05 à 0,8%, de préférence         0,20%
- Propylène glycol :
```

de 2,5 à 40%, de préférence          10,0%

- Conservateurs :

de 0,05 à 0,8%, de préférence        0,20%

- Eau déminéralisée :

qsp                     100%

- Parfum :

de 0,02 à 0,4%, de préférence       0,10%

- Poudre de noyaux d'abricots :

de 0,2 à 4,%, de préférence         1,00%

- Pétales broyés de bleuet, camomille, rose :

de 0,8 à 14%, de préférence :      3,50%

On utilise ce gel pour éliminer les cellules mortes de l'épiderme.

Exemple III : Emulsion adoucissante

- Huile minérale :

de 3 à 44%, de préférence          11%

- Octyl dodecanol oxyethylène (30) :

5 à 80%, de préférence            20%

- Polyethylèneglycol oléique :

de 1,5 à 24%, de préférence        6%

- Eau déminéralisée :

qsp                     100%

- Sorbitol 70% :

de 2 à 28%, de préférence         7,00%

- Propylène glycol :

de 1,2 à 20%, de préférence       5,00%

- Pétales broyés d'immortelle et camomille :

de 0,05 à 0,20%, de préférence    0,07%

Ce produit se présente sous la forme d'une émulsion transparente de pH compris entre 4,8 et 5,2 et ayant des propriétés émollientes.

Exemple IV : Gel vitaminique

- Poudre de racines de ginseng :

de 3 à 20%, de préférence         15,00%

- Sorbitol 70% :

30 à 80%, de préférence          71,00%

- Na-carraghenane :

0,08 à 1,2%, de préférence        0,30%

```
- Aromatisant :
   0,05 à 0,8%, de préférence          0,20%
- Colorant :                              qs
- Eau déminéralisée :              qsp 100%
```

Ce gel pourra être utilisé dans le cas de fatigue général, surmenage ou stress.

Les plantes ou fragments de plantes des exemples mentionnés ci-dessus sont préalablement traités par enrobage, selon le procédé décrit ci-dessous.

Il s'agit d'une méthode permettant de préserver la ou les plantes fraîches ou sèches, sous la forme intégrale ou broyée, au moyen d'une ou plusieurs couches protectrices pouvant, dans certains cas, avoir une action cosmétologique, pharmaceutique ou thérapeutique propre, ou pouvant faciliter l'utilisation de ladite ou desdites plantes dans un ou plusieurs milieux non propices.

La ou les diverses couches entrant dans la composition d'un enrobage, est ou sont généralement à base de charges minérales et/ou organiques telles que : oxyde de fer, oxyde de zinc, oxyde de titane, oxyde de chrome, de talc, de silice (adjuvants neutres et inertes), acides aminés, sucre, lécithine, soie, cholestérol (adjuvant humectant, hydratant et/ou traitant). Cette liste, bien entendu, n'est pas exhaustive.

L'enrobage est effectué soit par un ou plusieurs étalements successifs de solution que l'on assèche par dessication, étuvage, micro-ondes, soit par voie sèche.

En fonction de la nature du produit fini, de l'action recherchée du produit fini, et des vertus des plantes intégrales ou broyées utilisées, la forme de l'enrobage tient compte de la forme desdites plantes intégrales ou broyées.

Les avantages de l'enrobage sont multiples :

1 - Possibilité de préserver l'apparence des plantes intégrales ou broyées dans une composition de gels ou dans une émulsion.

2 - Préserver les vertus des plantes intégrales ou broyées utilisées.

3 - Augmenter les vertus des plantes intégrales ou broyées utilisées, par l'association des vertus de l'enrobage utilisé pour la cosmétologie, pharmacie et thérapeutique voulue.

Au lieu de traiter les plantes par enrobage, on peut, alternativement, les traiter par lyophilisation avant de les incorporer à la composition selon l'invention.

La lyophilisation est une technique de dessiccation par sublimation de la glace de solutions préalablement solidifées par congélation. La méthode, du fait même de son procédé : basse température, pression réduite, absence de phase liquide intermédiaire, permet d'éliminer les facteurs d'altération et de dénaturation des produits traités, intervenant à des degrés divers dans les techniques de dessiccation classique.

L'opération de lyophilisation comprend plusieurs étapes :

1 - Une congélation totale :

la plus rapide possible à une température nettement inférieure à celle du point d'eutexie le plus bas.

2 - Une sublimation qui exige :

- un fort abaissement de la pression à l'intérieur de l'enceinte contenant le produit congelé, selon le diagramme pression température des états de l'eau ;

- un apport contrôlé de calories pour compenser l'importante absorption de chaleur due au changement de phase ;

- une mise hors-circuit de la vapeur produite ;

- une élimination continue des gaz condensables.

3 - Une déshydratation finale :

Il s'agit d'une dessiccation complémentaire destinée à éliminer l'eau résiduelle fixée par phénomènes d'adsorption.

Cette méthode permet d'obtenir des produits secs de texture poreuse, de caractère lyophile, possédant une stabilité et une conservation fortement accrues.

## Revendications

**1 -** Composition à base de plantes, caractérisée en ce que les éléments solides sont obtenus à partir de plantes fraîches et/ou sèches, que ces éléments solides sont des plantes entières et/ou des parties entières de ces plantes et/ou des grains ou des particules de ces mêmes plantes ayant subi un traitement leur assurant une couche de protection.

**2 -** Composition selon la revendication 1, utilisable notamment en cosmétologie, en pharmacie ou en hygiène et comprenant un support transparent comportant un gel ou une émulsion.

**3 -** Composition selon la revendication 1, caractérisée en ce que la couche de protection comprend des charges minérales ou organiques, choisies dans le groupe comprenant :

- oxyde de fer ;
- oxyde de zinc ;
- oxyde de titane ;
- oxyde de chrome ;
- talc ;
- silice ;
- acides aminés ;
- sucres ;
- lécithine ;
- soie ;
- cholestérol.

**4 -** Composition selon l'une des revendications 1 à 3, caractérisée en ce que les éléments solides sont obtenus par lyophilisation de plantes ou fragments de plantes.

**5 -** Composition selon l'une des revendications 1 à 4, caractérisée en ce que ledit support constitue un milieu préservant toutes les vertus de ladite plante.

**6 -** Composition selon l'une des revendications précédentes, caractérisée en ce qu'elle contient de 0,05 à 20% en poids de plantes.

**7 -** Composition selon l'une des revendications précédentes, caractérisée en ce qu'elle contient de 0,1 à 21% en poids d'agents gélifiants.

**8 -** Composition selon l'une des revendications précédentes, caractérisée en ce qu'elle contient de 5 à 50% en poids d'agents tensioactifs.

**9 -** Composition selon l'une des revendications précédentes, caractérisée en ce que les plantes sont choisies dans le groupe comprenant :

- tamarin ;
- artichaut ;
- bourrache pour purifier les voies biliaires ;
- gentiane ;
- quinquina pour stimuler l'appétit ;
- fucus ;
- badiane ;
- mélisse pour faciliter la digestion ;
- coquelicot ;
- passiflore ;
- tilleul ;
- camomille pour traiter les troubles du sommeil, cannelle, églantier, maté, thé pour les asthénies fonctionnelles, états de fatigue passagers ;
- marron d'inde, petit houx, hamamélis, myrtille, cassis pour traitement de la circulation veineuse et capillaire ;
- artichaut, cerise, cassis, framboise pour faciliter n l'élimination urinaire ;
- vanille, frêne, sureau, thé comme adjuvant des régimes amaigrissants ;
- ginseng pour obtenir un produit à propriétés vitaminiques et apportant des éléments minéraux ;
- la camomille (Anthémis nobilis, Matricaria chamomilla) ;
- le bleuet (Centaurea cyanus) ;
- la rose (rosa rubra, rosa centifolia) ;
- la menthe (mentha aquatica L;, arvensis L., M. spicata var. crispa L., M. piperata L., M. viridis L.) ;
- la réglisse (Glycycrrhiza glabra, glycyrrhiza uranacis) ;
- immortelle (Helichrysum arenarium, Helichrysum staechas).

**10 -** Composition selon l'une des revendications précédentes, caractérisée en ce que l'agent gélifiant est choisi dans le groupe comprenant :

- les galactomannanes ;
- les farines à base de céréales alimentaires ;
- la cellulose et ses dérivés ;
- les acides polyacryliques réticulés ;
- les silices ;

- la gélatine ;
- l'agar agar ;
- les gommes adragantes ou arabiques ;
- les alginates et les pectines.

**11 -** Composition selon la revendication 7 à 10, caractérisée en ce que l'agent tensioactif est choisi dans le groupe comprenant les anioniques, les non-ioniques, les cationiques et les amphotères.

**12 -** Composition selon l'une des revendications précédentes, caractérisée en ce qu'elle comprend un véhicule de l'élément actif de la plante, contenant un ou plusieurs glycols.

**13 -** Composition selon l'une des revendications précédentes, utilisable en cosmétologie, caractérisée en ce qu'elle comprend l'un au moins des adjuvants cosmétiques suivants :
- modificateurs de pH ;
- parfums ;
- agents conservateurs ;
- Agents antioxydants;
- agents colorants.

**14 -** Composition selon l'une des revendications précédentes, caractérisée en ce qu'elle comprend :

```
- Polyethylèneglycol (30)
  nonyphenyl éther :          de 0,5  à     8%
- Polyethylèneglycol



  dodecyl éther :             de 0,25 à     4%
- α-bisabolol :              de 0,02 à   0,4%
- Conservateurs :            de 0,1  à   1,6%
- Butanediol-1,3 :           de 0,8  à    12%
- Acide alginique :          de 0,5  à    10%
- L-Lysine (sol. à 50% aq.): de 0,5  à     8%
- L-Arginine :               de 0,07 à   1,2%
- Aminobutanol :             de 0,05 à   0,8%
- Parfum :                   de 0,92 à   0,4%
- Pétales broyés de
  camomille et de menthe :   de 0,2  à     4%.
```

**15 -** Composition selon l'une des revendications 1 à 11, caractérisée en ce qu'elle comprend :

```
- Acide polyacrylique :      de 0,15 à 2,4%
- Gomme xanthane :           de 0,05 à 0,8%
- Propylène glycol :         de 2,5  à  40%
- Conservateurs :            de 0,05 à 0,8%
- Parfum :                   de 0,02 à 0,4%
- Poudre de noyaux
  d'abricots :               de 0,2  à   4%
- Pétales broyés de bleuts,
  camomille, rose :          de 0,8  à  14%.
```

**16 -** Composition selon l'une des revendications 1 à 13, caractérisée en ce qu'elle comprend :

```
- Huile minérale :              de    3    à    44%
- Octyl dodecanol
  oxyethylène (30) :            de    5    à    80%
- Polyethylèneglycol
  oléique:                      de 1,5    à    24%
- Sorbitol 70% :               de    2    à    28%
- Propylène glycol :           de 1,2    à    20%
- Pétales broyés
  d'immortelle et camomille: de 0,05   à    0,20%.
```

**17 -** Composition selon l'une des revendications 1 à 13, caractérisée en ce qu'elle comprend :

```
- Poudre de racines
  de ginseng                   de 3     à    20%
- Sorbitol 70% :              de 30     à    80%


- Na-carraghenane :           de 0,08   à    1,2%
- Aromatisant :               de 0,05   à    0,8%.
```

**18 -** Procédé de préparation d'une composition selon l'une des revendications précédentes, caractérisé en ce que l'on utilise comme produits de départ des plantes fraîches ou sèches, en ce qu'on les traite par un procédé leur assurant une bonne conservation dans le temps, puis on introduit les éléments solides ainsi obtenus dans un support transparent sous forme de gel ou d'émulsion, dont la viscosité permet le maintien en suspension desdits éléments solides.

**19 -** Procédé selon la revendication 18, caractérisé en ce que les plantes sont broyées, soit à température ambiante, soit à très basse température après avoir subi le traitement de conservation.

**20 -** Procédé selon la revendication 18 ou 19, caractérisé en ce que ledit procédé de conservation consiste en un enrobage des plantes.

EP 0 519 777 A1

Office européen
des brevets

**RAPPORT DE RECHERCHE EUROPEENNE**

Numero de la demande

EP 92 40 1468

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | US-A-4 735 814 (CHIGURUPATI)<br>* le document en entier * | 1 | A61K7/48<br>A61K35/78 |
| X | 'PETIT LAROUSSE ILLUSTRE'<br>1973 , LIBRAIRIE LAROUSSE , PARIS<br>* page 240 : "confire" * | 1,3 | |
| X,P | EP-A-0 449 717 (LABORATOIRES ARDEVAL)<br>* le document en entier * | 1-13 | |
| X | FR-A-2 622 453 (LABORATOIRE VAILLANT-DEFRESNE)<br>* en entier * | 1-13 | |
| A | S.T.N. Serveur de bases de donnees, Karlsruhe,DE<br>Fichier Chemical Abstracts vol. 110, no. 22528v<br>UCHIDA: 'Gelling of coffee and other plant<br>extracts with gellan gum '<br>* abrègé * | 1-13 | |
| A | EP-A-0 234 143 (LABORATOIRE VAILLANT-DEFRESNE)<br>* en entier * | 1-13 | DOMAINES TECHNIQUES RECHERCHES (Int. Cl.5) |
| A | LU-A-83 173 (L'OREAL)<br>* en entier * | 1-22 | A61K |
| A | PHYTOCHEMISTRY<br>vol. 24, no. 1, 1985, GRANDE BRETAGNE<br>pages 163 - 169;<br>H.G. THEUNS ET AL.: 'constituents of papaver bracteatum'<br>* page 164, colonne de gauche, dernier paragraphe * | 1 | |

Le présent rapport a été établi pour toutes les revendications

| Lieu de la recherche | Date d'achèvement de la recherche | Examinateur |
|---|---|---|
| LA HAYE | 16 SEPTEMBRE 1992 | FISCHER J.P. |